Europäisches Patentamt

European Patent Office

Office européen des brevets

(11) Veröffentlichungsnummer: **0 303 939**

**A1**

(12) **EUROPÄISCHE PATENTANMELDUNG**

(21) Anmeldenummer: 88112903.5

(22) Anmeldetag: 08.08.88

(51) Int. Cl.⁴: **A61N 1/08 , A61N 1/36**

(30) Priorität: 21.08.87 DE 3727971

(43) Veröffentlichungstag der Anmeldung:
22.02.89 Patentblatt 89/08

(84) Benannte Vertragsstaaten:
AT BE CH DE FR LI NL

(71) Anmelder: **Siemens Aktiengesellschaft Berlin
und München
Wittelsbacherplatz 2
D-8000 München 2(DE)**

(72) Erfinder: **Helmreich, Klaus
Rhoenstrasse 36
D-8520 Erlangen(DE)**
Erfinder: **Herzog, Ludwig
Drei-Thorn-Strasse 3
D-6948 Waldmichelbach(DE)**
Erfinder: **Knapp, Volker
Pestalozzistrasse 19
D-6948 Waldmichelbach(DE)**

(54) **Reizstromgerät für Konstantspannungsbetrieb.**

(57) Das Reizstromgerät umfaßt einen Spannungsgeber, dessen Spannung über eine Intensitätseinstelleinrichtung (3) an den Reizstromelektroden (8, 9) konstant anlegbar ist. Es beinhaltet ferner eine Strom-Sicherheitsschaltung (1, 10, 11, 12) mit einem Iststromwerterfasser (10) und einem Stromschwellwertgeber (12), der einen Stromschwellwert vorgibt, der so ausgelegt ist, daß bei kleinstmöglichem auftretenden Patientenwiderstand und bei geringer Spannungserhöhung von Null aus die Sicherheitsschaltung gerade noch nicht anspricht.

FIG 1

EP 0 303 939 A1

## Reizstromgerät für Konstantspannungsbetrieb

Die Erfindung bezieht sich auf ein Reizstromgerät für Konstantspannungsbetrieb gemäß Oberbegriff des Patentanspruchs 1.

Bei solchen Reizstromgeräten kann es bei Konstantspannungsbetrieb (CV) zu steilheitsbedingten Stromüberhöhungen kommen, wenn die eingestellte Steilheit der Reizimpulse groß ist, da der kapazitive Anteil des Patientenwiderstandes nicht vernachlässigbar ist. Dies führt dazu, daß die Anfangseinstellung der Konstantspannung gewöhnlich nicht fein genug bei Impulsen mit hoher Steilheit im Vergleich zu solchen mit niedriger Steilheit (z.B. Rechteckimpulse im Vergleich zu Dreiecksimpulsen) eingestellt werden kann. Darüber hinaus kann bei nicht vorhandener Feineinstellung von Null ausgehend nicht gewährleistet werden, daß es nicht zu unerwünschten Reizsensationen am Patienten kommt. Die einstellende Bedienungsperson muß also zur Feineinstellung gezwungen werden. Außerdem muß bei Konstantspannungsbetrieb vermieden werden, daß bereits unter Spannung stehende Elektroden beim Applizieren unerwünschte Reizsensationen hervorrufen. Dennoch muß bei hochohmigen Applikationen (hochohmiger Patientenwiderstand und/oder kleine Elektrodenflächen) eine hohe Anfangsspannung einstellbar sein, um überhaupt einen Stromfluß zu erhalten.

Herkömmliche Sicherheitsschaltungen außerhalb des CV-Betriebes, die das Gerät bei zu hohem Patientenstrom abschalten, sind zur Lösung der vorliegenden CV-Problematik nicht geeignet.

Aufgabe der vorliegenden Erfindung ist es also, eine Sicherheitsschaltung speziell für Konstantspannungsbetrieb anzugeben, die die Bedienungsperson zur Feineinstellung bei Impulsen mit großer Steilheit zwingt, und die dafür sorgt, daß es bei jeder Art von Elektrodenapplikation mit Sicherheit nicht zu Reizsensationen beim Patienten kommt.

Die Aufgabe wird erfindungsgemäß durch die kennzeichnenden Merkmale des Anspruchs 1 gelöst.

Die Erfindung zwingt die Bedienungsperson zur Feineinstellung bis zur Erststromerfassung. Stellt die Bedienungsperson nämlich nicht fein genug ein, d.h. wird die Spannung in der Anfangsphase zu rasch hochgestellt, dann erreicht der eingestellte Strom sehr rasch den Stromschwellwert und die Sicherheitsschaltung unterbricht den Patientenstromkreis. Die Bedienungsperson ist jetzt gezwungen erneut, diesmal jedoch feiner bzw. langsamer, einzustellen. Reizsensationen sind entsprechend nicht möglich.

In der Ausgestaltung der Erfindung gemäß Anspruch 2 ist gewährleistet, daß die Sicherheitsschaltung bei hohem Patientenwiderstand erst bei relativ hoher Spannung, die tatsächlich zu einem überhöhten Strom führt, anspricht und nicht schon zu Beginn einer Einstellung.

In der Ausgestaltung der Erfindung gemäß Anspruch 3 spricht jedoch die Sicherheitsschaltung an, wenn eine hohe Spannung an den Elektroden bereits anliegt, wenn diese appliziert werden.

Der Stromschwellwert liegt zweckmäßigerweise im Bereich 2 bis 10 mA, vorzugsweise bei 5 mA.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen

Fig. 1 die Erfindung im Prinzipschaltbild,

Fig. 2 bis 4 Impulsdiagramme.

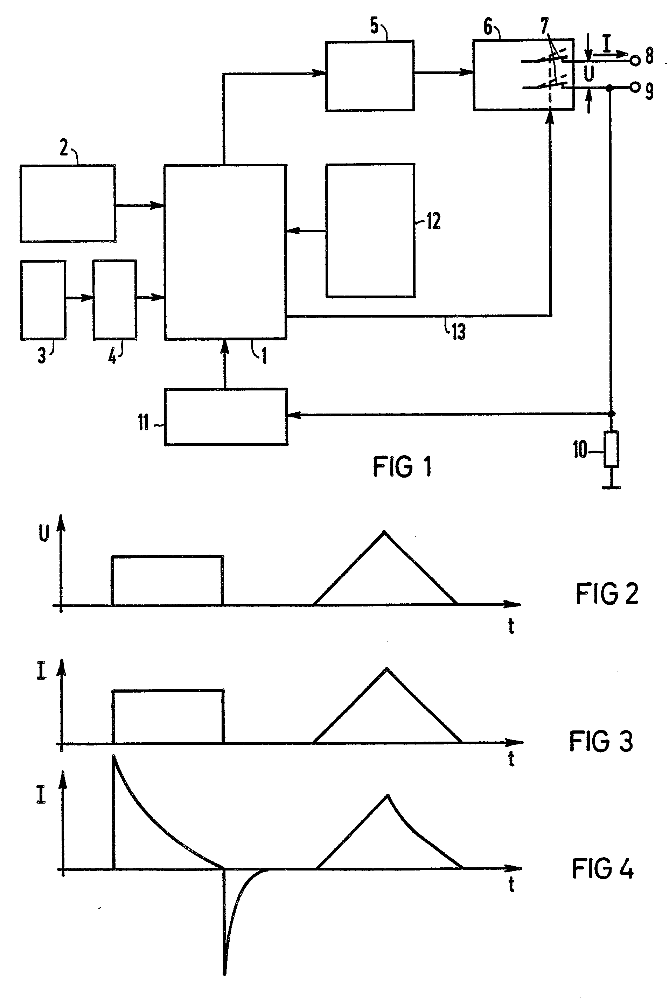

In der Fig. 1 wird einem zentralen Mikroprozessor 1 über eine Stromarteingabeeinrichtung 2 die Stromart bzw. über einen Intensitätssteller 3 (Potentiometer) mit nachgeschaltetem Analog-Digital-Wandler 4 die Intensität der Konstantspannung eingegeben. Der Mikroprozessor erzeugt über eine Impulsform- und Intensitätskarte 5 die entsprechend gewählte Reizstromimpulsform mit zugehöriger Intensität (Konstantspannung CV).

Die Reizstromimpulse werden über eine Endstufe 6 mit Patientenrelais 7 (schematisch dargestellt) den Reizelektroden 8, 9 zugeführt.

Der Istwert des Stromes wird an einem Widerstand 10 abgegriffen und über einen Analog-Digital-Wandler 11 dem Mikroprozessor 1 zugeführt. Der Block 12 umfaßt alle weiteren Steuer- und Speichereinheiten für den Mikroprozessor 1 zusammen. Mikroprozessor 1 und Steuer- und Speichereinheiten 12 bilden also zusammen eine Programmschaltgruppe.

Von der Steuer- und Speichereinheit 12 wird auch der Stromschwellwert gemäß der vorliegenden Erfindung dem Mikroprozessor zugeführt. Dieser vergleicht den zugeführten Stromschwellwert mit dem Istwert und erzeugt das Stromabschaltsignal auf der Leitung 13, wenn der Istwert den Schwellwert überschreitet. Der Schwellwert ist dabei in der Weise eingestellt, wie es in den Patentansprüchen beansprucht ist.

Die Fig. 2 bis 4 zeigen Impulsdiagramme für die zwischen den Reizelektroden 8, 9 angelegte Spannung U in Abhängigkeit von der Zeit t (Fig. 2) und sich dabei ergebende Reizstromformen I in Abhängigkeit von der Zeit t bei reellem Patientenwiderstand (Fig. 3) und komplexem Patientenwiderstand (Fig. 4). Wäre der Patientenwiderstand immer reell, so würden, wie in der Fig. 3 dargestellt ist, die Stromimpulse streng dem zeitlichen Verlauf der Spannungsimpulse U(t) folgen. Gewöhnlich ist jedoch der Patientenwiderstand komplex, d.h. er umfaßt insbesondere eine kapazitive Komponente. Das Ergebnis ist die Stromimpulsform I(t) gemäß Fig. 4. Es ergeben sich also insbesondere bei steilen Impulsflanken (Rechteckimpuls) der Spannung starke Stromüberhöhungen im Bereich der steilen Flanken (Anstiegs- bzw. Abfallflanken). Bei langsamen Anstiegs- bzw. Abfallzeiten der Impulse (z.B. Spannungsdreiecksimpuls gemäß Fig. 2) kommt es hingegen kaum zu Stromerhöhungen. Damit verursachen also insbesondere rechteckförmige oder annähernd rechteckförmige Spannungsimpulse U(t) die eingangs beschriebenen Probleme, die durch die vorliegende erfindungsgemäße Sicherheitsschaltung gelöst werden.

**Ansprüche**

1. Reizstromgerät für Konstantspannungsbetrieb, mit einem Spannungsgeber, dessen Spannung über eine Intensitätseinstelleinrichtung an den Reizstromelektroden konstant anlegbar ist, **gekennzeichnet durch** eine Stromsicherheitsschaltung (1, 10, 11, 12) mit einem Iststromwerterfasser (10) und einem Stromschwellwertgeber (12), der einen Stromschwellwert vorgibt, der so ausgelegt ist, daß bei kleinstmöglichem auftretenden Patientenwiderstand und bei geringer Spannungserhöhung von Null aus die Sicherheitsschaltung gerade noch nicht anspricht.

2. Reizstromgerät nach Anspruch 1, **dadurch gekennzeichnet,** daß der Stromschwellwert auch so gewählt ist, daß bei eingestellter hoher Spannung an einem hohen Patientenwiderstand die Sicherheitsschaltung bei Stromerstwerterfassung noch nicht anspricht.

3. Reizstromgerät nach Anspruch 1 oder 2, **dadurch gekennzeichnet,** daß der Stromschwellwert auch so gewählt ist, daß, wenn bei bereits eingestellter Spannung die Reizstromelektroden (8, 9) appliziert werden, bei einem Reizstrom unterhalb des Stromschwellwertes die Reizschwelle des Patienten nicht überschritten wird.

4. Reizstromgerät nach einem der Ansprüche 1 bis 3, **dadurch gekennzeichnet,** daß der Stromschwellwert im Bereich 2 bis 10 mA gewählt ist.

5. Reizstrom nach Anspruch 4, **dadurch gekennzeichnet,** daß der Stromschwellwert zu 5 mA gewählt ist.

87 P 8559

FIG 1

FIG 2

FIG 3

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 216 911 (DATRON-ELECTRONIC) * Seite 16, Zeile 3 - Seite 22, Zeile 2; Seite 23, Zeilen 1-12; Seite 32, Zeile 21 - Seite 33, Zeile 4; Figuren 1-3,8 * | 1 | A 61 N 1/08 A 61 N 1/36 |
| A | | 2-5 | |
| P,X | EP-A-0 270 828 (SIEMENS) * Insgesamt * | 1 | |
| A | FR-A-2 500 689 (FAIVELEY) * Seite 1, Zeilen 29-32 * | 1,4,5 | |
| A | EP-A-0 087 617 (SIEMENS) * Seite 8, Zeilen 15-29; Seite 9, Zeilen 26-31; Anspruch 6; Figur 3 * | 1-5 | |
| A | US-A-4 177 819 (KOFSKY) * Spalte 4, Zeilen 3-24; Figuren 1,5 * | 1 | |

**RECHERCHIERTE SACHGEBIETE (Int. Cl.4)**

A 61 N

Der vorliegende Recherchenbericht wurde für alle Patentansprüche erstellt

| Recherchenort | Abschlußdatum der Recherche | Prüfer |
|---|---|---|
| DEN HAAG | 21-11-1988 | SCHMIERER U.J. |